# EUROPEAN PATENT SPECIFICATION

(11) **EP 3 134 074 B1**
(45) Date of publication and mention of the grant of the patent: **03.06.2020**
(21) Application number: 15783867.3
(22) Date of filing: 24.04.2015
(51) Int. Cl.: A61K 9/00, A61K 9/06, A61K 47/40, A61K 47/42, A61L 27/24, A61L 27/54, A61P 27/02

(54) **COMPOSITIONS COMPRISING CYCLODEXTRIN INCORPORATED COLLAGEN MATRICES FOR USE IN BIOMEDICAL APPLICATIONS**
ZUSAMMENSETZUNGEN MIT CYCLODEXTRININTEGRIERTEN KOLLAGENMATRIZEN ZUR VERWENDUNG IN BIOMEDIZINISCHEN ANWENDUNGEN
COMPOSITIONS COMPRENANT DES MATRICES DE COLLAGÈNE INCORPORANT DES CYCLODEXTRINES, DESTINÉES À UNE UTILISATION DANS DES APPLICATIONS BIOMÉDICALES

(30) Priority: 25.04.2014 US 201461984328 P
(43) Date of publication of application: 01.03.2017
(73) Proprietor: The Johns Hopkins University, Baltimore, MD 21218 (US)
(72) Inventor: ELISSEEFF, Jennifer, Baltimore, Maryland 21218 (US); GUO, Qiongyu, Bethlehem, PA 18015 (US); MAJUMDAR, Shoumyo, Baltimore, Maryland 21218 (US)
(74) Representative: Murgitroyd & Company
(86) International application number: PCT/US2015/027503
(87) International publication number: WO 2015/164733

(56) References cited:
- WO-A1-2013/040559
- US-B2- 7 297 348
- CALDERÓN-COLÓN XIOMARA ET AL: "Structure and properties of collagen vitrigel membranes for ocular repair and regeneration applications", BIOMATERIALS, vol. 33, no. 33, 21 August 2012 (2012-08-21), pages 8286-8295, XP028938893, ISSN: 0142-9612, DOI: 10.1016/J.BIOMATERIALS.2012.07.062
- CHELLAM J ET AL: "Influence of cyclodextrins on the physical properties of collagen", INTERNATIONAL JOURNAL OF PHARMA AND BIO SCIENCES OCTOBER/DE, vol. 4, no. 4, October 2013 (2013-10), pages 795-806, XP009501608, ISSN: 0975-6299
- HASSELL, J. R. ET AL.: 'The molecular basis of corneal transparency' EXPERIMENTAL EYE RESEARCH vol. 91, no. 3, 2010, ISSN 0014-4835 pages 326 - 335, XP055233188
- GUO, Q. ET AL.: 'Modulation of keratocyte phenotype by collagen fibril nanoarchitecture in membranes for corneal repair' BIOMATERIALS vol. 34, no. 37, 2013, ISSN 0014-4835 pages 9365 - 9372, XP055233188
- DAVIS, M. E. ET AL.: 'Cyclodextrin-based pharmaceutics: past, present and future' NATURE REVIEWS DRUG DISCOVERY vol. 3, no. 12, 2004, ISSN 1474-1776 pages 1023 - 1035, XP002613123
- GUO, Q. ET AL.: 'Developing biomimetic collagen-based matrix using cyclodex trin for corneal repair' NORTHEAST BIOENGINEERING CONFERENCE (NEBEC 25 April 2014, pages 1 - 2, XP032692798

## Description

### REFERENCE TO RELATED APPLICATIONS

This application claims the benefit of U.S. Provisional Patent Application No. 61/984,328, filed on April 25, 2014.

### STATEMENT OF GOVERNMENTAL INTEREST

This invention was made with government support under contract no. W81XWH-09-2-0173 awarded by the U.S. Army. The government has certain rights in the invention.

### BACKGROUND OF THE INVENTION

Extracellular matrix (ECM) is a complex mixture of macromolecules consisting of proteins, proteoglycans, and other soluble molecules. Collagen, the most abundant protein in animals, is widely applied in various bioapplications. However, little effort has been made on engineering an ECM scaffold composed of both collagen and proteoglycans due to difficulty of deriving large amount of purified proteoglycans. In native cornea, the proteoglycans play a critical role in corneal transparency by regulating the collagen fibril diameter and spacing. Therefore, finding a proteoglycan substitute to develop biomimetic collagen-based ECM is a promising line of research that will provide a new template to solve the challenging issues associated with clinical applications, such as corneal regeneration.

Cyclodextrins (CDs) are a family of cyclic oligomers composed of a ring of six to eight glucose molecules. These ring molecules feature an inner hydrophobic core and an outer hydrophilic ring that can form complexes with small molecules or portions of large compounds. The solubility of natural cyclodextrins is very poor and initially this prevented cyclodextrins from becoming effective complexing agents. In the late 1960's, it was discovered that chemical substitutions at the 2-, 3-, and 6-hydroxyl sites would greatly increase solubility. The degree of chemical substitution and the nature of the groups used for substitution determine the final maximum concentration of cyclodextrin in an aqueous medium. Most chemically modified cyclodextrins are able to achieve a 50% (w/v) concentration in water.

### SUMMARY OF THE INVENTION

In accordance with one or more embodiments, the inventors hypothesized that, similar to proteoglycan in native tissues, the incorporation of cyclodextrins in collagen matrix would regulate collagen fibrogenesis while preserving collagen triple helical formation. Traditional engineered type I collagen matrices with fibrillar architectures are opaque, whereas transparent gels composed of amorphous collagen networks exhibit poor mechanical properties. It was expected that cyclodextrins could be added to a collagen matrix as a proteoglycan substitute and would assist in optimizing both optical and mechanical properties for corneal regeneration.

In accordance with an embodiment, the present invention provides a composition comprising a vitrified matrix gel having a first component and a second component, wherein the first component comprises collagen, and wherein the second component comprises cyclodextrin.

In accordance with another embodiment, the present invention provides a composition comprising a vitrified matrix gel having a first component and a second component, wherein the first component comprises collagen, and wherein the second component comprises cyclodextrin, and further comprises at least one biologically active agent.

In accordance with a further embodiment, the present invention provides a method for making a vitrified matrix gel having a first component and a second component, wherein the first component comprises collagen, and wherein the second component comprises cyclodextrin, comprising: a) obtaining an aqueous solution of collagen; b) obtaining an aqueous solution of cyclodextrin; c) combining the solutions of a) and b); and d) dehydrating the combined solution of c) for a period of time sufficient to allow vitrification of the solution.

In accordance with a yet another embodiment, the present invention provides the use of the compositions described above as a matrix for repair of a tissue of a mammal.

In accordance with another embodiment, the present invention provides the use of the compositions described above as a matrix for repair of the cornea of an eye of a mammal.

### BRIEF DESCRIPTION OF THE DRAWINGS

Figure 1 depicts differential scanning calorimetry (DSC) first heating curve of three collagen-based membranes, including normal collagen vitrigel (CV), α-CD-col composition of the present invention, and crosslinked CV.
Figure 2 is a graph showing that the α-CD-col composition of the present invention exhibited an excellent transparency with a transmittance of as high as 96% at 550 nm. An inset in the figure shows a photograph of a wet membrane placed on a printed word "eye".
Figure 3 is a graph depicting Load vs. displacement of a suturablity test of two α-CD-col compositions of the present invention with a thickness of 520 µm and 170 µm, respectively. An inset in the figure shows a photograph of the thicker membrane after strentched over 5.6 mm.
Figure 4 is a photograph of the apparatus used in the load test of the compositions of the present invention.
Figure 5 shows a schematic of cell protrusion analysis and the effect of different vitrigel compositon collagen density of primary cultures of bovine keratocytes.
Figure 6 depicts how gene expression of three different gene markers in primary cultures of bovine keratocytes is affected by the fibril nanoarchitecture of the vitrigel compositions of the present invention.
Figure 7 is a schematic showing the architecture of various cyclodextrins and how they interact with collagen fibrils in solution. The spaces in the cyclodextrin molecules can be used as drug reservoirs for water insoluble drugs and biologically active agents.

### DETAILED DESCRIPTION OF THE INVENTION

In accordance with one or more embodiments, the present inventors employed cyclodextrins for use as a proteoglycan substitute to engineer a biomimetic collagen-based matrix composition. The resulting incorporation of cyclodextrin in the inventive collagen compositions increased collagen thermal stability and reduced collagen fibrogenesis. As a result, a thick, transparent and mechanically strong collagen-based composition was formed. This cyclodextrin-collagen composition holds a great potential to be used as a therapeutic eye patch for corneal repair.

In accordance with an embodiment, the present invention provides a composition comprising a vitrified matrix gel having a first component and a second component, wherein the first component comprises collagen, and wherein the second component comprises cyclodextrin.

As used herein, the term "vitrification" or "vitrigel" means that the composition is composed of an aqueous solution of a mixture of one or more collagens and one or more cyclodextrins and allowed to form a hydrogel. In some embodiments, the gelation of the composition is performed at a temperature of 37 °C. After the hydrogel is formed, the hydrogel is vitrified by dehydration, such as, for example, heating the hydrogel at a specific temperature and humidity, for a specific length of time to allow vitrification to occur. In some embodiments, the vitrification is performed at a temperature of 35 to 45 °C and a humidity of between about 30% and 50% relative humidity. In an embodiment, the vitrification is performed at a temperature of 40 °C and a relative humidity of 40%. The time needed for vitrification of the compositions can vary from a few days to a few weeks. In an embodiment, the time for vitrification of the compositions is about 1 to 2 weeks.

"Gel" refers to a state of matter between liquid and solid, and is generally defined as a cross-linked polymer network swollen in a liquid medium. Typically, a gel is a two-phase colloidal dispersion containing both solid and liquid, wherein the amount of solid is greater than that in the two-phase colloidal dispersion referred to as a "sol." As such, a "gel" has some of the properties of a liquid (i.e., the shape is resilient and deformable) and some of the properties of a solid (i.e., the shape is discrete enough to maintain three dimensions on a two-dimensional surface).

By "hydrogel" is meant a water-swellable polymeric matrix that can absorb water to form elastic gels, wherein "matrices" are three-dimensional networks of macromolecules held together by covalent or noncovalent crosslinks. On placement in an aqueous environment, dry hydrogels swell by the acquisition of liquid therein to the extent allowed by the degree of cross-linking.

The compositions of the present invention comprise collagen. The collagen of the first component is selected from the group consisting of Type I, Type II, Type III and Type IV collagen. In an embodiment, the collagen used as the first component of the composition is Type I collagen. One of ordinary skill in the art would understand that the collagen used in the compositions and methods could include more than one type of collagen.

The compositions of the present invention also comprise cyclodextrins. The cyclodextrin of the second component is selected from the group consisting selected from the group consisting of α-cyclodextrin, β-cyclodextrin, and γ-cyclodextrin. In an embodiment, the cyclodextrin used as the second component of the composition is α-cyclodextrin. One of ordinary skill in the art would understand that the cyclodextrin used in the compositions and methods could include more than one type of cyclodextrin.

As used herein, the vitrified compositions of the present invention are hydrated prior to use.

The vitrigel compositions of the present invention are optically transparent and suitable for a variety of uses. In one embodiment the vitrigel composition has an optical transparency of about 96% at 550 nm.

It will be understood that the vitrigel compositions of the present invention can be molded or formed into any particular shape suitable for use as a replacement tissue or tissue filler. The instant invention provides for ex vivo polymerization techniques to form scaffolds and so on that can be molded to take the desired shape of a tissue defect, promote tissue development by stimulating native cell repair, and can be potentially implanted by minimally invasive injection.

In one embodiment, the vitrigel compositions of the present invention can be shaped for use as an artificial cornea for a subject.

In accordance with another embodiment, the present invention provides a composition comprising a vitrified matrix gel having a first component and a second component, wherein the first component comprises collagen, and wherein the second component comprises cyclodextrin, and further comprises at least one biologically active agent.

An "active agent" and a "biologically active agent" are used interchangeably herein to refer to a chemical or biological compound that induces a desired pharmacological and/or physiological effect, wherein the effect may be prophylactic or therapeutic. The terms also encompass pharmaceutically acceptable, pharmacologically active derivatives of those active agents specifically mentioned herein, including, but not limited to, salts, esters, amides, prodrugs, active metabolites, analogs and the like. When the terms "active agent," "pharmacologically active agent" and "drug" are used, then, it is to be understood that the invention includes the active agent per se as well as pharmaceutically acceptable, pharmacologically active salts, esters, amides, prodrugs, metabolites, analogs etc.

Incorporated," "encapsulated," and "entrapped" are art-recognized when used in reference to a therapeutic agent, dye, or other material and a polymeric composition, such as a composition of the present invention. In certain embodiments, these terms include incorporating, formulating or otherwise including such agent into a composition that allows for sustained release of such agent in the desired application. The terms may contemplate any manner by which a therapeutic agent or other material is incorporated into a matrix, including, for example, distributed throughout the matrix, appended to the surface of the matrix (by intercalation or other binding interactions), encapsulated inside the matrix, etc. The term "co-incorporation" or "co-encapsulation" refers to the incorporation of a therapeutic agent or other material and at least one other therapeutic agent or other material in a subject composition.

In one aspect of this invention, a composition comprising a vitrigel composition and one or more biologically active agents may be prepared. The biologically active agent may vary widely with the intended purpose for the composition. The term active is art-recognized and refers to any moiety that is a biologically, physiologically, or pharmacologically active substance that acts locally or systemically in a subject. Examples of biologically active agents, that may be referred to as "drugs", are described in well-known literature references such as the Merck Index, the Physicians' Desk Reference, and The Pharmacological Basis of Therapeutics, and they include, without limitation, medicaments; vitamins; mineral supplements; substances used for the treatment, prevention, diagnosis, cure or mitigation of a disease or illness; substances which affect the structure or function of the body; or pro-drugs, which become biologically active or more active after they have been placed in a physiological environment. Various forms of a biologically active agent may be used which are capable of being released by the vitrigel composition, for example, into adjacent tissues or fluids upon administration to a subject. In some embodiments, a biologically active agent may be used to, for example, treat, ameliorate, inhibit, or prevent a disease or symptom, in conjunction with, for example, the eye.

Non-limiting examples of biologically active agents include following: adrenergic blocking agents, anabolic agents, androgenic steroids, antacids, anti-asthmatic agents, antiallergenic materials, anti-cholesterolemic and anti-lipid agents, anti-cholinergics and sympathomimetics, anti-coagulants, anti-convulsants, anti-diarrheal, anti-emetics, antihypertensive agents, anti-infective agents, anti-inflammatory agents such as steroids, non-steroidal anti-inflammatory agents, anti-malarials, anti-manic agents, anti-nauseants, antineoplastic agents, anti-obesity agents, anti-parkinsonian agents, anti-pyretic and analgesic agents, anti-spasmodic agents, anti-thrombotic agents, anti-uricemic agents, anti-anginal agents, antihistamines, anti-tussives, appetite suppressants, benzophenanthridine alkaloids, biologicals, cardioactive agents, cerebral dilators, coronary dilators, decongestants, diuretics, diagnostic agents, erythropoietic agents, estrogens, expectorants, gastrointestinal sedatives, agents, hyperglycemic agents, hypnotics, hypoglycemic agents, ion exchange resins, laxatives, mineral supplements, mitotics, mucolytic agents, growth factors, neuromuscular drugs, nutritional substances, peripheral vasodilators, progestational agents, prostaglandins, psychic energizers, psychotropics, sedatives, stimulants, thyroid and anti-thyroid agents, tranquilizers, uterine relaxants, vitamins, antigenic materials, and prodrugs.

Various forms of the biologically active agents may be used. These include, without limitation, such forms as uncharged molecules, molecular complexes, salts, ethers, esters, amides, prodrug forms and the like, which are biologically activated when implanted, injected or otherwise placed into a subject.

The vitrigel compositions will be formulated, dosed and administered in a manner consistent with good medical practice. Factors for consideration in this context include the particular disorder being treated, the particular mammal being treated, the clinical condition of the individual patient, the cause of the disorder, the site of delivery of the agent, the method of administration, the scheduling of administration, and other factors known to medical practitioners. The "therapeutically effective amount" of the biopolymer to be administered will be governed by such considerations, and can be the minimum amount necessary to prevent, ameliorate or treat a disorder of interest. As used herein, the term "effective amount" is an equivalent phrase refers to the amount of a therapy (e.g., a prophylactic or therapeutic agent), which is sufficient to reduce the severity and/or duration of a disease, ameliorate one or more symptoms thereof, prevent the advancement of a disease or cause regression of a disease, or which is sufficient to result in the prevention of the development, recurrence, onset, or progression of a disease or one or more symptoms thereof, or enhance or improve the prophylactic and/or therapeutic effect(s) of another therapy (e.g., another therapeutic agent) useful for treating a disease.

In one embodiment, the repair of damaged tissue may be carried out within the context of any standard surgical process allowing access to and repair of the tissue, including open surgery and laparoscopic techniques. Once the damaged tissue is accessed, a vitrigel composition of the invention is placed in contact with the damaged tissue along with any surgically acceptable patch or implant, if needed.

The present disclosure further provides a method for making a vitrified matrix gel having a first component and a second component, wherein the first component comprises collagen, and wherein the second component comprises cyclodextrin, comprising: a) obtaining an aqueous solution of collagen; b) obtaining an aqueous solution of cyclodextrin; c) combining the solutions of a) and b); and d) dehydrating the combined solution of c) for a period of time sufficient to allow vitrification of the solution.

As used herein, the aqueous solution of collagen is any collagen solution dissolved in a suitable buffer. The concentration of the collagen is variable, however solutions of collagen with a concentration in a range of 1 mg/ml to about 10 mg/ml can be used with the methods of the present invention. In an embodiment, the concentration of collagen in aqueous solution is about 5 mg/ml.

As used herein, the aqueous solution of cyclodextrin is any cyclodextrin solution dissolved in a suitable buffer. The concentration of the cyclodextrin is variable, however solutions of cyclodextrin with a concentration in a range of 2.5 mg/ml to about 10 mg/ml can be used with the methods of the present invention.

In accordance with the disclosed methods the dehydration and vitrification of the composition of the present invention comprises drying the solution comprising the collagen solution and cyclodextrin at a temperature of about 5 to 40 °C, at a relative humidity of between about 30 to 50%, and for a time of about 3 days to about 28 days. In an example of the disclosure, vitrification of the composition of the present invention comprises heating the solution comprising the collagen solution and cyclodextrin at a temperature of 39 °C for about 7 days. In another example of the disclosure, vitrification of the composition of the present invention comprises heating the solution comprising the collagen solution and cyclodextrin at a temperature of 39 °C for about 14 days.

The term, "carrier," refers to a diluent, adjuvant, excipient or vehicle with which the therapeutic is supplied with the vitrigel composition of the present invention. Such physiological carriers can be sterile liquids, such as water and oils, including those of petroleum, animal, vegetable or synthetic origin, such as peanut oil, soybean oil, mineral oil, sesame oil and the like. Water is a suitable carrier when the pharmaceutical composition is administered intravenously. Saline solutions and aqueous dextrose and glycerol solutions also can be employed as liquid carriers, particularly for injectable solutions. Suitable pharmaceutical excipients include starch, glucose, lactose, sucrose, gelatin, malt, rice, flour, chalk, silica gel, sodium stearate, glycerol monostearate, talc, sodium chloride, dried skim milk, glycerol, propylene glycol, water, ethanol and the like. The composition, if desired, can also contain minor amounts of wetting or emulsifying agents, or pH buffering agents.

Buffers, acids and bases may be incorporated in the compositions to adjust pH. Agents to increase the diffusion distance of agents released from the composition may also be included.

Buffering agents help to maintain the pH in the range which approximates physiological conditions. Buffers are preferably present at a concentration ranging from about 2 mM to about 50 mM. Suitable buffering agents for use with the instant invention include both organic and inorganic acids, and salts thereof, such as citrate buffers (e.g., monosodium citrate-disodium citrate mixture, citric acid-trisodium citrate mixture, citric acid-monosodium citrate mixture etc.), succinate buffers (e.g., succinic acid monosodium succinate mixture, succinic acid-sodium hydroxide mixture, succinic acid-disodium succinate mixture etc.), tartrate buffers (e.g., tartaric acid-sodium tartrate mixture, tartaric acid-potassium tartrate mixture, tartaric acid-sodium hydroxide mixture etc.), fumarate buffers (e.g., fumaric acid-monosodium fumarate mixture, fumaric acid-disodium fumarate mixture, monosodium fumarate-disodium fumarate mixture etc.), gluconate buffers (e.g., gluconic acid-sodium glyconate mixture, gluconic acid-sodium hydroxide mixture, gluconic acid-potassium gluconate mixture etc.), oxalate buffers (e.g., oxalic acid-sodium oxalate mixture, oxalic acid-sodium hydroxide mixture, oxalic acid-potassium oxalate mixture etc.), lactate buffers (e.g., lactic acid-sodium lactate mixture, lactic acid-sodium hydroxide mixture, lactic acid-potassium lactate mixture etc.) and acetate buffers (e.g., acetic acid-sodium acetate mixture, acetic acid-sodium hydroxide mixture etc.). Phosphate buffers, carbonate buffers, histidine buffers, trimethylamine salts, such as Tris, HEPES and other such known buffers can be used.

Preservatives may be added to retard microbial growth, and may be added in amounts ranging from 0.2%-1 % (w/v). Suitable preservatives for use with the present invention include phenol, benzyl alcohol, m-cresol, octadecyldimethylbenzyl ammonium chloride, benzyaconium halides (e.g., chloride, bromide and iodide), hexamethonium chloride, alkyl parabens, such as, methyl or propyl paraben, catechol, resorcinol, cyclohexanol and 3-pentanol.

Isotonicifiers are present to ensure physiological isotonicity of liquid compositions of the instant invention and include polhydric sugar alcohols, preferably trihydric or higher sugar alcohols, such as glycerin, erythritol, arabitol, xylitol, sorbitol and mannitol. Polyhydric alcohols can be present in an amount of between about 0.1 % to about 25%, by weight, preferably 1% to 5% taking into account the relative amounts of the other ingredients.

Examples of diluents include a phosphate buffered saline, buffer for buffering against gastric acid in the bladder, such as citrate buffer (pH 7.4) containing sucrose, bicarbonate buffer (pH 7.4) alone, or bicarbonate buffer (pH 7.4) containing ascorbic acid, lactose, or aspartame. Examples of carriers include proteins, e.g., as found in skim milk, sugars, e.g., sucrose, or polyvinylpyrrolidone. Typically these carriers would be used at a concentration of about 0.1-90% (w/v) but preferably at a range of 1-10%

The formulations to be used for in vivo administration must be sterile. That can be accomplished, for example, by filtration through sterile filtration membranes. For example, the formulations of the present invention may be sterilized by filtration.

### EXAMPLES

Type I collagen-based membranes incorporated with different cyclodextrins were prepared following a three-stage sequence: gelation, vitrification and rehydration. Three cyclodextrins were tested and compared, i.e. α-CD, β-CD and γ-CD.
First, a type I collagen solution (5 mg/ml) was quickly and thoroughly mixed at 1:1 v/v ratio with 2% HEPES solution containing CD (at a range of concentrations of 2.5 to about 10 mg/ml). The mixed solution was gelled at 37 °C and 5% CO₂ for 2 hours.
Second, the CD-collagen (CD-col) gels were vitrified in a humidifier at about 39 °C and relative humidity of about 40% for one week.
Third, these col-CD membranes were rehydrated in water or buffered solution for at least 2 hours before usage. Two collagen membranes, i.e. collagen vitrigel and crosslinked vitrigel, were prepared as controls following the procedures as previously described (Biomaterials 34 (2013) 9365-9372). Compared to normal vitrigel, the crosslinked vitrigel was fabricated with additional 0.6% 1-ethyl-3-(3-dimethylaminopropyl) carbodiimide (EDC) and 0.6% N-hydroxysuccinimide (NHS) in the collagen media mixture before the gelation process. The specific interactions between collagen and CD were evaluated using differential scanning calorimetry (DSC, Perkin Elmer, Waltham, MA). Absorbance of CD-col membranes was measured using a Synergy 2 microplate reader (BioTek, Winooski, VT). Membrane suturability was tested with a 10-0 nylon suture using an Electroforce 3200 testing instrument (Bose, Eden Prairie, MN).

Keratocytes were isolated from full-thickness corneas using a sequential collagenase (Type 2, Worthington Biochemical Corp., Lakewood, NJ) digestion. The digested cells were collected and centrifuged at 1400 rpm for 10 minutes. The cell pellet was resuspended and cultured on general tissue culture plates (TCP) or CV-covered plates at 37 °C and 5% CO₂. Either serum-free or serum based culture medium was used. The serum-free medium consisted of DMEM/F-12, 1% of 10 U/mL penicillin-streptomycin, and 0.5% of 1.25 mg/mL amphotericin B (Life Technologies, Carlsbad, CA). The serum-based medium contained DMEM/F-12, 10% FBS, 1% of 10 U/mL penicillin-streptomycin, and 0.5% of 1.25 mg/mL amphotericin B. The cells were plated at a concentration of 5000 cells/cm² using the serum-free medium, while at a concentration of 1000 cells/cm² using the serum-based medium. Before imaging and gene expression analyses, keratocytes were cultured over 3 wk in serum-free medium or 6 d in serum-based medium. Keratocyte morphologies were examined by staining with the LIVE/DEAD® Viability/Cytotoxicity Kit (Life Technologies). Indomethacin was encapsulated in the vitrigels by soaking the vitrified membranes in 0.1% indomethacin eye drops for a period of time. The elution of indomethacin from the vitrigel was measured using high-performance liquid chromatography (HPLC). The release solution was tested using a mobile phase of acetonitrile:water of 51:49 (v/v), a C18 column and a UV/VIS detector set at 318 nm.

### EXAMPLE 1

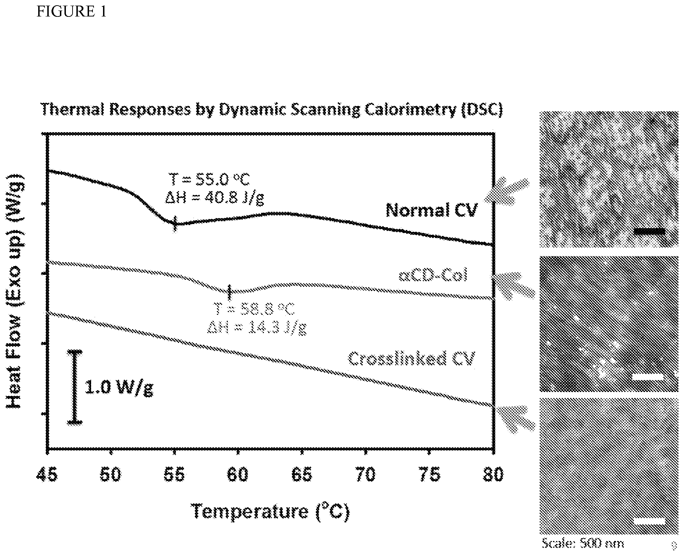

All three cyclodextrins, especially α-CD, exhibited strong interactions with type I collagen triple helices, leading to formation of transparent and mechanically strong CD-col membranes. As shown in Figure 1, normal vitrigel exhibited a large and broad endothermic peak at 55 °C in the heat flow, which indicates that the collagen membrane underwent a thermal denaturation with an enthalpy of 40.8 J/g. In contrast, no discernible peak was found in the control sample of crosslinked vitrigel, suggesting a denatured feature of the collagen membrane due to the crosslinking reaction. Compared to normal vitrigel, the addition of a-CD in collagen membrane led to an increased denaturation temperature, indicating an enhanced thermal stability. Only a single narrower peak was observed in α-CD-col, which means that the matrix had a homogeneous structure. If we assume that all of the enthalpy comes from the thermal transition of collagen triple helices, the denaturation enthalpy of a-CD-col was 70.1% of that from normal vitrigel, suggesting a reduced collagen fibrogenesis. Similar results were also observed in β-CD-col and γ-CD-col.

### EXAMPLE 2

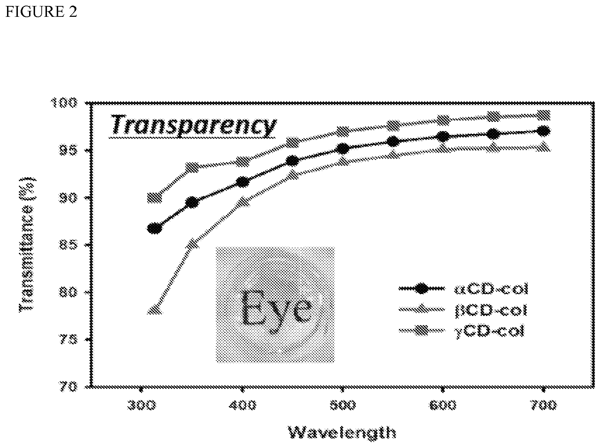

Compared to conventional collagen membrane, the CD-col membranes showed greatly enhanced transparency (Figure 2), which can be explained by the reduced collagen fibrogenesis.

### EXAMPLE 3

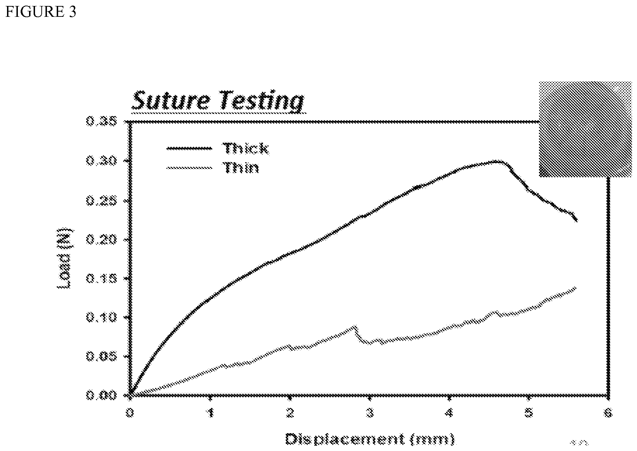

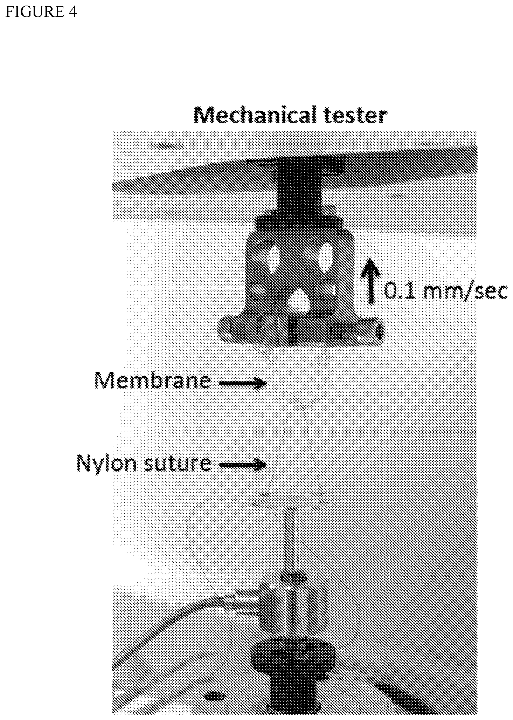

The inventive compositions demonstrated superior mechanical properties. When their thickness was comparable to that of human cornea (i.e. ∼500 µm), they became strong enough for suture. As shown in Figure 3, a nylon suture was pulling through a hole in α-CD-col membrane with a thickness of 520 µm. Under the stress of suture, the hole in the thick membrane was only stretched, instead of tearing through the membrane as observed in the thin one with a thickness of 170 µm (Fig. 4).

### EXAMPLE 4

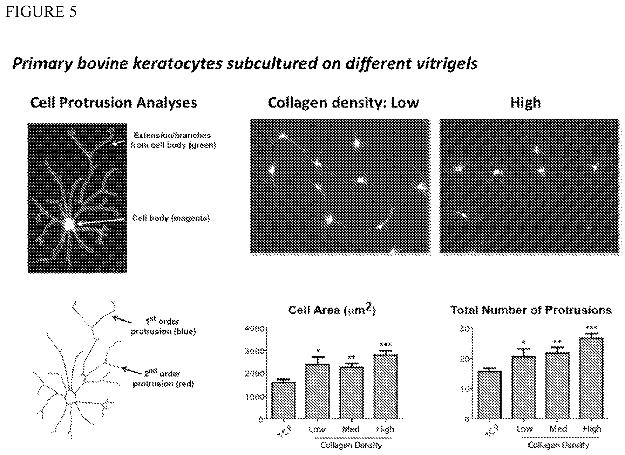

Collagen nanoarchitecture defines cell response. Primary cultures of bovine keratocytes were cultured on vitrigel compositions having low and high collagen density. As shown in Figure 5, collagen density of the compositions of the present invention allowed the keratocytes to have greater protrusion area in culture when compared with normal vitrigel controls. In addition, the total number of cell protrusions of the keratocytes were significantly increased as a function of the collagen density of the inventive vitrigel compositions were increased (Fig. 5).

### EXAMPLE 5

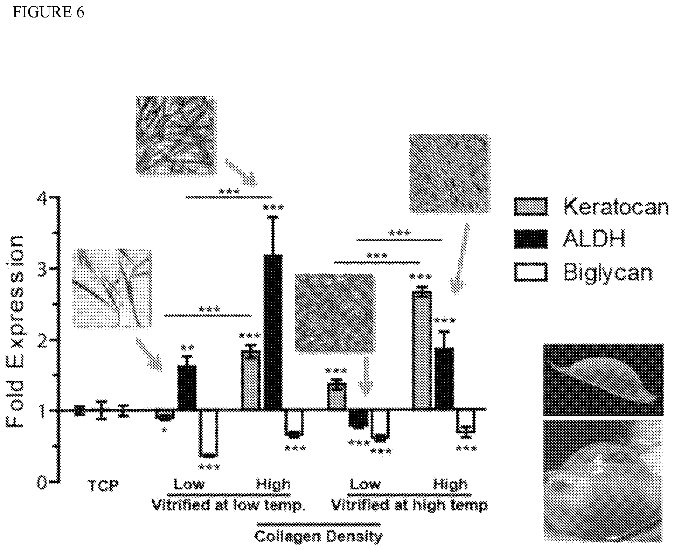

Keratocyte gene expression is dependent on fibril architecture. Keratocytes were cultured on control vitrigels or with the inventive vitrigel compositions where the vitrigels were dehydrated at 5 °C or 39 °C temperatures. After growth for 6 days in serum-based medium, the cells were harvested and analyzed for gene expression of keratocan, aldehyde dehydrogenase (ALDH) and biglycan. The expression of these genes was analyzed by isolating total RNA from cultured keratocytes using TRIzol reagent (Life Technologies), reverse transcribing into cDNA using SuperScript II First Strand Synthesis Kit (Life Technologies) and then testing the cDNA using real-time PCR reactions on a StepOnePlus Real-Time PCR System (Applied Biosystems®, Life Technologies). As shown in Figure 6, when compared with controls, the gene expression of keratocan and ALDH was greatly increased when the cells were grown on the vitrigel compositions dehydrated at high temperature. Expression of biglycan was reduced in the vitrigel compositions when compared to controls at both low and high temperatures.

### EXAMPLE 6

The vitrigel compostions of the present invention can be used to deliver biologically active agents. The vitrigel compositions were prepare as above, and a solution of a commercially available eye drop formulation of 0.1% indomethacin in ethanol was added to the composition for either 10 minutes using two drops or overnight soaking in 1 mL eye drop after hydrating the vitrigels, and the release kinetics were tested using HPLC. It was found that the vitrigel composition released the indomethacin from the vitrigel composition over a 5 hour period.

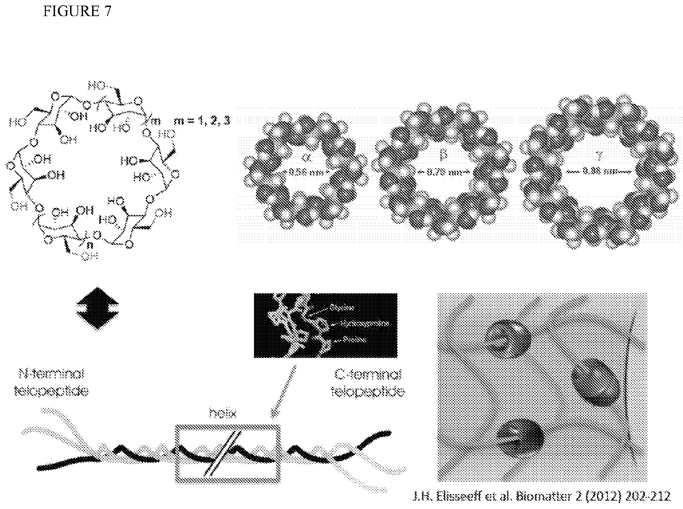

Type I collagen-CD compositions of the present invention were developed with optimized optical and mechanical properties for corneal regeneration. While not being limited to any particular theory, these properties are probably due to regulated collagen fibrillogenesis in the cyclodextrin-incorporated collagen compositions (Fig. 7). These inventive compositions hold a great potential to be used as therapeutic eye patch for corneal repair and treatments. The compositions and methods disclosed herein may also be useful for regeneration of other connective tissues derived from fibril-forming collagens, such as cartilage, skin and blood vessel.

The use of the terms "a" and "an" and "the" and similar referents in the context of describing the invention (especially in the context of the following claims) are to be construed to cover both the singular and the plural, unless otherwise indicated herein or clearly contradicted by context. The terms "comprising," "having," "including," and "containing" are to be construed as open-ended terms (i.e., meaning "including, but not limited to,") unless otherwise noted. Recitation of ranges of values herein are merely intended to serve as a shorthand method of referring individually to each separate value falling within the range, unless otherwise indicated herein, and each separate value is incorporated into the specification as if it were individually recited herein. All methods described herein can be performed in any suitable order unless otherwise indicated herein or otherwise clearly contradicted by context. The use of any and all examples, or exemplary language (e.g., "such as") provided herein, is intended merely to better illuminate the invention and does not pose a limitation on the scope of the invention unless otherwise claimed. No language in the specification should be construed as indicating any non-claimed element as essential to the practice of the invention.

Preferred embodiments of this invention are described herein, including the best mode known to the inventors for carrying out the invention. Variations of those preferred embodiments may become apparent to those of ordinary skill in the art upon reading the foregoing description.

## Claims

1. A composition comprising a vitrified matrix gel having a first component and a second component, wherein the first component comprises collagen, and wherein the second component comprises cyclodextrin.

2. The composition of claim 1, wherein the cyclodextrin of the second component is selected from the group consisting of α-cyclodextrin, β-cyclodextrin, and γ-cyclodextrin.

3. The composition of claim 1 or 2, wherein the collagen of the first component is selected from the group consisting of Type I, Type II, Type III and Type IV collagen.

4. The composition of claim 3, wherein the composition further comprises at least one biologically active agent.

5. The composition of claim 4, wherein the composition is hydrated.

6. The composition of claim 3, wherein the composition is formed into a shape suitable for use as an artificial cornea.

7. The composition of claim 3, wherein the composition has an optical transparency of above 90% at 550 nm.

8. The composition of claim 1, wherein the first component is Type I collagen and the second component is α-cyclodextrin, and the composition is formed into a shape suitable for use as an artificial cornea.

9. The composition of claim 1, wherein the first component is Type I collagen and the second component is α-cyclodextrin, and the composition is formed with a thickness of approximately 500 µm.

10. The composition of any of claims 1 to 9 for use as a medicament.

11. The composition of any of claims 1 to 9 for use as a matrix for repair of a tissue of a mammal.

12. The composition of any of claims 1 to 9 for use as a matrix for repair of a cornea of an eye of a mammal.

## Patentansprüche

1. Eine Zusammensetzung, beinhaltend ein vitrifiziertes Matrixgel, das eine erste und eine zweite Komponente aufweist, wobei die erste Komponente Kollagen beinhaltet, und wobei die zweite Komponente Cyclodextrin beinhaltet.

2. Zusammensetzung gemäß Anspruch 1, wobei das Cyclodextrin der zweiten Komponente ausgewählt ist aus der Gruppe, bestehend aus α-Cydodextrin, β-Cyclodextrin und γ-Cyclodextrin.

3. Zusammensetzung gemäß Anspruch 1 oder 2, wobei das Kollagen der ersten Komponente ausgewählt ist aus der Gruppe, bestehend aus Kollagen des Typs I, Typs II, Typs III und Typs IV.

4. Zusammensetzung gemäß Anspruch 3, wobei die Zusammensetzung ferner mindestens einen biologisch aktiven Wirkstoff beinhaltet.

5. Zusammensetzung gemäß Anspruch 4, wobei die Zusammensetzung hydratisiert ist.

6. Zusammensetzung gemäß Anspruch 3, wobei die Zusammensetzung in eine Form gebracht ist, die zur Verwendung als eine künstliche Hornhaut geeignet ist.

7. Zusammensetzung gemäß Anspruch 3, wobei die Zusammensetzung eine optische Transparenz von über 90 % bei 550 nm aufweist.

8. Zusammensetzung gemäß Anspruch 1, wobei die erste Komponente Kollagen des Typs I ist und die zweite Komponente α-Cydodextrin ist und die Zusammensetzung in eine Form gebracht ist, die zur Verwendung als eine künstliche Hornhaut geeignet ist.

9. Zusammensetzung gemäß Anspruch 1, wobei die erste Komponente Kollagen des Typs I ist und die zweite Komponente α-Cydodextrin ist und die Zusammensetzung mit einer Dicke von etwa 500 µm geformt ist.

10. Zusammensetzung gemäß einem der Ansprüche 1 bis 9 zur Verwendung als Medikament.

11. Zusammensetzung gemäß einem der Ansprüche 1 bis 9 zur Verwendung als eine Matrix zum Reparieren eines Gewebes eines Säugetiers.

12. Zusammensetzung gemäß einem der Ansprüche 1 bis 9 zur Verwendung als eine Matrix zum Reparieren einer Hornhaut eines Auges eines Säugetiers.

## Revendications

1. Une composition comprenant un gel de matrice vitrifié ayant un premier composant et un deuxième composant, où le premier composant comprend du collagène, et où le deuxième composant comprend de la cyclodextrine.

2. La composition de la revendication 1, où la cyclodextrine du deuxième composant est sélectionnée dans le groupe constitué d'a-cyclodextrine, de β-cydodextrine, et de γ-cyclodextrine.

3. La composition de la revendication 1 ou de la revendication 2, où le collagène du premier composant est sélectionné dans le groupe constitué de collagène de Type I, de Type II, de Type III et de Type IV.

4. La composition de la revendication 3, où la composition comprend en sus au moins un agent biologiquement actif.

5. La composition de la revendication 4, où la composition est hydratée.

6. La composition de la revendication 3, où la composition est façonnée en une forme convenant pour une utilisation comme cornée artificielle.

7. La composition de la revendication 3, où la composition a une transparence optique de plus de 90 % à 550 nm.

8. La composition de la revendication 1, où le premier composant est du collagène de Type I et le deuxième composant est de l'a-cyclodextrine, et la composition est façonnée en une forme convenant pour une utilisation comme cornée artificielle.

9. La composition de la revendication 1, où le premier composant est du collagène de Type I et le deuxième composant est de l'a-cyclodextrine, et la composition est façonnée avec une épaisseur d'approximativement 500 µm.

10. La composition de n'importe lesquelles des revendications 1 à 9 pour une utilisation comme médicament.

11. La composition de n'importe lesquelles des revendications 1 à 9 pour une utilisation comme matrice pour la réparation d'un tissu chez un mammifère.

12. La composition de n'importe lesquelles des revendications 1 à 9 pour une utilisation comme matrice pour la réparation d'une cornée d'un œil chez un mammifère.
